(19) **Europäisches Patentamt**
**European Patent Office**
**Office européen des brevets**

(11) **EP 3 512 573 B1**

(12) **FASCICULE DE BREVET EUROPEEN**

(45) Date de publication et mention
de la délivrance du brevet:
**02.06.2021 Bulletin 2021/22**

(21) Numéro de dépôt: **17780481.2**

(22) Date de dépôt: **15.09.2017**

(51) Int Cl.:
*A61L 27/38* *(2006.01)* *C12N 5/00* *(2006.01)*
*C12N 5/095* *(2010.01)* *A61L 27/36* *(2006.01)*
*C12N 5/071* *(2010.01)* *C12N 5/073* *(2010.01)*
*C12N 5/0775* *(2010.01)* *C12N 5/074* *(2010.01)*

(86) Numéro de dépôt international:
**PCT/FR2017/052472**

(87) Numéro de publication internationale:
**WO 2018/051035 (22.03.2018 Gazette 2018/12)**

(54) **PROCÉDÉ DE PRÉPARATION D'UNE COMPOSITION POUR LA RÉPARATION TISSULAIRE**

VERFAHREN ZUR HERSTELLUNG EINER ZUSAMMENSETZUNG ZUR GEWEBEREPARATUR

METHOD FOR PRODUCING A COMPOSITION FOR TISSUE REPAIR

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **15.09.2016 FR 1658656**

(43) Date de publication de la demande:
**24.07.2019 Bulletin 2019/30**

(73) Titulaire: **Symbioken**
**31100 Toulouse (FR)**

(72) Inventeur: **SANTRAN, Véronique**
**31170 Tournefeuille (FR)**

(74) Mandataire: **Ipside**
**6, Impasse Michel Labrousse**
**31100 Toulouse (FR)**

(56) Documents cités:
**US-A1- 2010 167 398     US-A1- 2010 330 047**

• **GRAYSON W L ET AL: "Hypoxia enhances
proliferation and tissue formation of human
mesenchymal stem cells", BIOCHEMICAL AND
BIOPHYSICAL RESEARCH COMMUNICATIONS,
ELSEVIER, AMSTERDAM, NL, vol. 358, no. 3, 6
juillet 2007 (2007-07-06), pages 948-953,
XP025322402, ISSN: 0006-291X, DOI:
10.1016/J.BBRC.2007.05.054 [extrait le
2007-05-26]**
• **GRAYSON WARREN L ET AL: "Effects of hypoxia
on human mesenchymal stem cell expansion and
plasticity in 3D constructs", JOURNAL OF
CELLULAR PHYSIOLOGY, WILEY
SUBSCRIPTION SERVICES, INC, US, vol. 207, no.
2, 1 mai 2006 (2006-05-01), pages 331-339,
XP002602209, ISSN: 0021-9541, DOI:
10.1002/JCP.20571 [extrait le 2005-12-05]**
• **PILGAARD L ET AL: "Transcriptional signature
of human adipose tissue-derived stem cells
(hASCs) preconditioned for chondrogenesis in
hypoxic conditions", EXPERIMENTAL CELL
RESEARCH, ELSEVIER, AMSTERDAM, NL, vol.
315, no. 11, 1 juillet 2009 (2009-07-01), pages
1937-1952, XP002602212, ISSN: 0014-4827, DOI:
10.1016/J.YEXCR.2009.01.020 [extrait le
2009-02-02]**

**EP 3 512 573 B1**

**Description**

**Domaine de l'invention**

La présente invention vise le domaine de la réparation de sites tissulaires biologiques.

Plus particulièrement, la présente invention s'inscrit dans le domaine de la préparation de compositions biologiques destinées à être injectées dans des sites tissulaires ayant subi des dommages, afin de les réparer.

L'invention concerne plus précisément un procédé de préparation d'une telle composition, une composition susceptible d'être obtenue par un tel procédé et son utilisation pour la réparation de tissus biologiques chez le mammifère, ainsi qu'un kit pour la mise en œuvre dudit procédé de préparation.

**État de l'art**

Il a été proposé par l'art antérieur des compositions à base de cellules souches destinées à être injectées au niveau de sites tissulaires lésés de mammifères pour la réparation tissulaire.

Les cellules souches sont une population possédant la capacité d'auto-renouvellement, une viabilité à long terme, et un caractère multipotent, ce qui s'avère très intéressant pour la réparation tissulaire.

Des matrices extracellulaires biocompatibles sont utilisées en tant que support pour les cellules souches lors de leur injection en vue de la réparation de sites tissulaires biologiques. Ces matrices ont pour effet de soutenir la greffe et le développement des cellules souches au sein du tissu biologique lésé (« Cavernous nerve repair with allogenic adipose matrix and autologous adipose-derived stem cells » Lin et al., Urology. Juin 2011 ; 77(6): 1509.e1-1509.e8. doi:10.1016/j.urology.2010.12.076).

En tant que cellules souches comme source pour la réparation tissulaire, il est notamment proposé par l'art antérieur d'utiliser des cellules souches embryonnaires. Des cellules souches adultes (par exemple, les cellules souches mésenchymateuses extraites de la moelle osseuse ou MSC pour « *mesenchymal stem* cells » en terminologie anglo-saxonne) ont été également proposées en tant que source alternative. Initialement identifiées comme une source de cellules ostéoprogénitrices, les MSC se différencient en adipocytes, chondrocytes, ostéoblastes et myoblastes in vitro et peuvent subir une différenciation in vivo ce qui les place au rang de candidats prometteurs pour la réparation de défauts mésodermique et la gestion de certaines maladies. D'un autre côté, l'utilisation clinique de ces MSC issues de la moelle osseuse présente des problèmes liés à la douleur, la morbidité et au faible nombre de cellules lors de leur isolation. Cela a conduit de nombreux chercheurs à chercher d'autres sources de cellules souches mésenchymateuses.

Le tissu adipeux, comme la moelle osseuse, est d'origine mésodermique, il peut être facilement isolé. Le tissu adipeux contient notamment des préadipocytes et des cellules souches mésenchymateuses adultes (les préadipocytes et MSC étant différents) qui formeront des adipocytes en fonction de la balance énergétique, des conditions hormonales et de nutrition, ainsi que de l'environnement tissulaire dans lequel elles se trouvent. D'autres types cellulaires sont présents (fibroblastes, macrophages, cellules sanguines et endothéliales) et cet ensemble de cellules constitue la fraction du stroma-vasculaire du tissu adipeux (le stroma, au contraire du parenchyme, n'est pas spécifique du tissu). Sur cette base, le tissu adipeux représente une source de cellules souches mésenchymateuses que l'on nomme AMSC pour « *adipose mesenchymal stem cells* » en terminologie anglo-saxonne. Ces AMSC, de par leur potentiel, peuvent avoir des effets intéressants dans différents domaines.

En particulier, il a été identifié une population de cellules souches putatives au sein de tissus humains obtenus par liposuccion (Zuk et al., 2002, Mol Biol Cell. 2002 Dec;13(12):4279-95). Cette population peut être isolée à partir de tissu adipeux en quantité importante et présente une cinétique de croissance et de prolifération stable en culture. En outre, ces cellules ont la capacité de se différencier in vitro vers la lignée ostéogénique, adipogénique, myogénique et chondrogénique, lorsqu'elles sont traitées avec certains facteurs propres à la lignée établie. La capacité de différenciation multilignée de ces cellules a conduit à constater qu'une population de cellules AMSC, comparables aux cellules MSC connues, peut être isolée à partir de tissu adipeux humain. Un moyen d'obtention de cellules AMSC par extraction à partir de tissus adipeux pouvant provenir de différentes régions du corps humain (crural, abdominal, genou,...) est décrit dans la publication scientifique « Human Adipose Tissue Is a Source of Multipotent Stem Cells » de P. Zuk, 2002.

Concernant les matrices extracellulaires biocompatibles utilisées en tant que support pour les cellules souches, l'art antérieur enseigne l'utilisation de matrices extracellulaires synthétiques telles que des matrices de collagène et d'élastine, ou bien de matrices extracellulaires issues de tissus biologiques, par exemple de tissu adipeux.

Cependant, lors de tentatives de réparation tissulaire utilisant des cellules souches combinées à de telles matrices extracellulaires, il apparait un problème d'adhésion des cellules auxdites matrices et par voie de conséquence, de survie cellulaire, empêchant ainsi une réparation efficace des lésions tissulaires. Une solution à ce problème proposée par l'art antérieur est d'ajouter des peptides d'adhésion à la matrice. Cependant, ces derniers ont un coût important et leur mise en œuvre est en outre délicate à effectuer, en partie du point de vue de leur dosage, le risque étant de provoquer un problème lié à une trop forte adhésion des cellules souches dans l'organisme.

2

Dans la demande de brevet US 2010/330047 est divulguée une méthode visant à améliorer la capacité de prolifération de cellules MSC et/ou leur différentiation en un ou plusieurs types de cellules mésenchymateuses. Il s'agit d'une méthode comportant une première étape de réalisation d'une culture cellulaire comportant une pluralité de cellules MSC, une deuxième étape de traitement hypoxique de ces cellules MSC suivie d'une troisième étape de traitement normoxique (concentration normale d'oxygène) des cellules MSC. Les cellules MSC sont ensuite intégrées dans une matrice synthétique, par exemple de collagène ou fibrine, pour leur implantation dans les tissus d'un patient.

La publication scientifique intitulée *"hypoxia enhances prolifération and tissue formation of human mesenchymal stem cells"*, de GRAYSON W. L. & al., divulgue en outre que des cellules souches mésenchymateuses humaines maintiennent leur taux de croissance même après avoir atteint la confluence, tout en maintenant leur capacité de différenciation en différents types cellulaires. Cette publication montre également que les conditions hypoxiques affectent différents paramètres physiologiques des cellules souches comprenant la croissance et le développement in vitro.

Ainsi, on constate que la capacité de prolifération de cellules MSC et/ou leur différentiation est améliorée par leur traitement en condition hypoxique mais une amélioration plus importante et plus durable serait bénéfique.

La présente invention vise, entre autres, à remédier à ces inconvénients, en proposant un procédé permettant d'obtenir une composition adaptée à la réparation tissulaire qui favorise efficacement l'adhésion, la prolifération et la différenciation des cellules souches au niveau de sites tissulaires à réparer, de sorte à effectuer cette réparation de manière efficace. Des objectifs supplémentaires de l'invention sont que ce procédé soit simple, rapide et peu coûteux à mettre en œuvre.

## Exposé de l'invention

À cet effet, selon un premier aspect, la présente invention vise un procédé de préparation d'une composition destinée à être injectée au niveau d'un tissu biologique pour une réparation tissulaire, par mélange de cellules souches et d'une matrice choisie parmi une matrice extracellulaire obtenue à partir d'une portion de tissu biologique isolée de l'organisme biologique dont elle est issue, et une matrice synthétique biocompatible hydrophile, de charge positive et poreuse. Selon ce procédé, ladite matrice est soumise à un traitement par hypoxie avant ledit mélange.

Les cellules souches peuvent être des cellules souches embryonnaires et/ou des cellules souches adultes (post natal).

Les cellules souches embryonnaires utilisées dans la présente invention peuvent être choisies parmi les cellules souches totipotentes, multipotentes et pluripotentes. Ces cellules souches embryonnaires sont de préférence des cellules souches ayant été obtenues par une technique non destructive de l'embryon.

Dans des modes de mise en œuvre de l'invention, la matrice est mélangée à des cellules souches, à l'exclusion de cellules souches embryonnaires totipotentes.

Parmi les cellules souches adultes on considère notamment pour la présente invention les cellules souches hématopoïétiques (HSC pour *« hematopoietic stem cells »* en terminologie anglo-saxonne) et les cellules souches mésenchymateuses (MSC pour *« mesenchymal stem cells »* en terminologie anglo-saxonne). Les MSC peuvent par exemple être des cellules souches mésenchymateuses de tissu adipeux (AMSC pour *« adipose mesenchymal stem cells »* en terminologie anglo-saxonne), des cellules souches mésenchymateuses de moelle osseuse (BMSC pour *« bone marrow mesenchymal stem cells »* en terminologie anglo-saxonne) ou d'autres cellules souches mésenchymateuses extraites de pulpe dentaire, de bulbe capillaire, et bien entendu un mélange de deux ou plus de ces MSC.

Les cellules souches utilisées pour le procédé objet de la présente invention, peuvent également être des cellules souches pluripotentes induites (IPS pour *« Induced pluripotent stem cells »* en terminologie anglo-saxonne). Ces cellules souches IPS sont des cellules souches adultes modifiées génétiquement pour redevenir des cellules souches embryonnaires pluripotentes.

Toute combinaison des cellules souches différentes citées précédemment est envisageable pour la mise en œuvre du procédé selon la présente invention.

On entend, par « matrice extracellulaire », une matrice extracellulaire naturelle (issue d'un tissu biologique).. Les matrices naturelles comportent notamment le groupe des matrices cellularisées, le groupe des matrices décellularisées, et le groupe des matrices partiellement décellularisées. On entend par « matrice synthétique biocompatible », une matrice de synthèse biologique ou de synthèse chimique (par exemple de collagène et/ou d'élastine). La matrice synthétique biocompatible est hydrophile et de charge positive de sorte que les cellules souches puissent y adhérer. Elle est également poreuse de sorte que les fluides et échanges gazeux puissent avoir lieux en son sein et présente ainsi une grande surface spécifique.

Avantageusement, la matrice fournit des propriétés structurales favorisant la repopulation par les cellules de ladite matrice, en donnant la capacité d'organisation spatiale des cellules souches influençant leur adhésion et différenciation. La matrice influence de manière significative la morphologie et le phénotype cellulaires. La matrice ayant subi un traitement hypoxique engendre elle-même une hypoxie dans les cellules souches avec lesquelles elle est mise en contact ce qui favorise alors l'adhésion desdites cellules à ladite matrice. Elle favorise donc également la prolifération,

3

la survie et la différenciation des cellules souches et cela, en régulant en particulier les hétérodimères HIF-1α et HIF-2α (« *Hypoxia-inducible factor 1-alpha»* et « *Hypoxia-inducible factor 2-alpha »* en terminologie anglo-saxonne). La matrice ainsi traitée, en plus d'un rôle de support, présente alors avantageusement un effet de stimulation des cellules souches avec lesquelles elle est injectée au niveau d'un site tissulaire à réparer.

Plus précisément, les cellules souches intégrées à une telle matrice traitée par hypoxie sont stimulées pour s'orienter vers différentes voies de différenciation cellulaire propres à l'origine de ladite matrice (par exemple la voie de différenciation endothéliale vasculaire ou la voie de différenciation en kératinocytes), grâce à la stimulation des facteurs angiogéniques. Les cellules souches sont également stimulées pour s'orienter vers différentes voies de différenciation cellulaire propres à l'origine du tissu biologique dans lequel elles sont injectées avec la matrice. Ces cellules souches peuvent alors à leur tour participer au bon développement d'autres types cellulaires contenus dans le tissu biologique traité. Il y a donc une double orientation des cellules souches injectées : une orientation directe, grâce au microenvironnement en place, et une orientation indirecte, grâce au traitement spécifique de la matrice.

Par exemple, dans le cas d'une réparation tissulaire du derme, des cellules souches mélangées à la matrice traitée par hypoxie, puis injectées au niveau d'une lésion du derme, peuvent s'orienter à la fois vers la voie dermofibroblaste et vers la voie micro vasculaire endothéliale, pour une véritable régénération du derme. La vascularisation est notamment un processus indispensable à toute régénération de tissu et particulièrement de la peau.

Dans un mode de mise en œuvre particulier du procédé, les cellules souches sont soumises à un traitement par hypoxie avant le mélange.

Au sein des cellules souches, l'hypoxie entraine une augmentation du métabolisme énergétique, de l'angiogenèse avec sécrétion de facteurs angiogéniques tels que le facteur de croissance de l'endothélium vasculaire (VEGF ou « *Vascular endothelial growth factor »* en terminologie anglo-saxonne), l'interleukine 6 (IL-6), le facteur de croissance des fibroblastes FGF-2, le facteur de croissance 1 ressemblant à l'insuline (IGF-1 ou « *Insulin-like growth factor-1 »* en terminologie anglo-saxonne), le facteur de croissance des hépatocytes (HGF ou « *Hepatocyte growth factor »* en terminologie anglo-saxonne) et de manière tout à fait avantageuse dans le contexte de la présente invention, une augmentation de l'adhésion des cellules souches dans les tissus *in vivo.*

Dans un mode de mise en œuvre particulier du procédé, le traitement de la matrice par hypoxie comprend une étape de mise en présence de ladite matrice avec une solution destinée à générer une hypoxie de ladite matrice, dite solution hypoxique. Dans un mode de mise en œuvre particulier, la mise en présence de la matrice avec la solution hypoxique est réalisée sous agitation. Dans un autre mode de mise en œuvre, cette étape est réalisée sans agitation.

La solution hypoxique peut être de type contenant de la deferoxamine (DFX) comprenant un ion fer chélateur, ou tout autre agent générant l'hypoxie, par exemple, le protoporphyrine de Cobalt.

Dans un mode de mise en œuvre particulier, la solution hypoxique est une solution comprenant du chlorure de cobalt ($CoCl_2$). La concentration en $CoCl_2$ de ladite solution est de préférence comprise entre 40 µM et 350 µM, de préférence comprise entre 50 µM et 200 µM. La durée de mise en présence de la matrice avec la solution de $CoCl_2$ peut être de 15 minutes à plusieurs jours.

La mise en présence de la matrice avec la solution hypoxique peut être suivie d'une étape de centrifugation de ladite matrice de sorte à séparer la matrice de la solution hypoxique. On élimine alors la fraction aqueuse (comprenant la solution hypoxique) issue de la centrifugation.

Alternativement, le traitement de la matrice par hypoxie peut comprendre une étape de placement de ladite matrice dans un incubateur hypoxique avec pré-équilibration du milieu de l'incubateur en condition hypoxique. De telles conditions hypoxiques sont par exemple : 2% de dioxygène ($O_2$), 5% de dioxyde de carbone ($CO_2$) et 93% de diazote ($N_2$). Selon un tel exemple de conditions hypoxiques, la matrice est placée dans l'incubateur hypoxique pendant au moins 4 heures.

Dans un mode de mise en œuvre particulier, le procédé comprend une étape préalable d'extraction desdites cellules souches à partir d'une portion de tissu adipeux isolée d'un organisme humain ou animal. On obtient ainsi des cellules souches mésenchymateuses de tissu adipeux (AMSC). Les cellules AMSC ont un effet angiogénique avantageusement plus marqué que les cellules souches mésenchymateuses de la moelle osseuse.

Dans un mode de mise en œuvre particulier, le procédé comprend une étape préalable d'obtention de la matrice extracellulaire à partir d'une portion de tissu biologique isolée d'un organisme humain ou animal, ledit tissu biologique étant du même type que le tissu biologique pour la réparation tissulaire duquel ladite composition est destinée à être utilisée.

On entend par « du même type » le fait que le tissu biologique de la portion isolée provient d'un organe similaire à celui comportant le tissu biologique à réparer. La composition préparée selon l'invention peut servir à la réparation et/ou régénération de différents tissus biologiques (derme, cartilage, tissu nerveux, etc.). Le choix du tissu biologique dont est isolée une portion en vue de l'obtention de la matrice extracellulaire, qui conditionne le type de matrice extracellulaire (osseuse, tendineuse,...), se fait en fonction du tissu biologique receveur (à réparer).

Par exemple, si le tissu biologique à réparer est de type osseux, le procédé selon l'invention met en œuvre une portion de tissu biologique isolée de tissu osseux. Ainsi est obtenue une matrice extracellulaire osseuse selon cette étape préalable du procédé objet de la présente invention. De même, si le tissu biologique à réparer est de type peau, le procédé selon l'invention met en œuvre une portion de tissu biologique isolée de tissu adipeux. Ainsi est obtenue une matrice extracellulaire adipeuse selon cette étape préalable du procédé objet de la présente invention.

Ainsi, selon un mode de mise en œuvre particulièrement préféré de la présente invention, la portion de tissu biologique isolée est une portion de tissu adipeux.

Dans un tel exemple de réalisation particulier où la matrice extracellulaire est obtenue à partir d'une portion de tissu adipeux isolée d'un organisme humain ou animal, il est possible d'obtenir également des cellules souches, à partir de la même portion de tissu adipeux isolée. A cet effet, selon un mode de mise en œuvre de la présente invention, le procédé comprend une étape d'extraction des cellules souches à partir de la même portion de tissu adipeux servant à l'obtention de la matrice extracellulaire, ladite étape d'extraction des cellules souches et l'étape d'obtention de la matrice extracellulaire comprenant de préférence une même étape initiale de digestion de ladite portion de tissu adipeux isolée. Ensuite intervient une séparation de la fraction comportant la matrice extracellulaire et de la fraction comportant les cellules, chaque fraction étant ensuite soumise à un traitement pour obtenir respectivement la matrice extracellulaire et les cellules souches du tissu adipeux prêtes pour l'étape de mélange avant injection. Il s'agit là de cellules souches mésenchymateuses du tissu adipeux. Ce mode de mise en œuvre représente un gain de temps pour l'obtention de la composition issue du procédé de l'invention.

Préférentiellement, la portion de tissu biologique isolée provient de l'individu même pour lequel la composition issue du procédé selon l'invention est destinée à être mise en œuvre pour la réparation tissulaire. Cela permet d'éviter, de façon avantageuse, les problèmes de cytocompatibilité/biocompatibilité de la matrice, lorsque ladite composition est injectée au niveau d'une lésion d'un tissu biologique d'un individu. Les cellules souches embryonnaires n'étant pas immunogènes et les cellules souches adultes étant faiblement immunogènes, elles ne posent avantageusement pas ce problème de biocompatibilité.

Pour obtenir la matrice extracellulaire à partir d'une portion de tissu biologique isolée, selon un mode de mise en œuvre particulièrement préféré du procédé selon l'invention, il est réalisé une digestion de ce tissu. La digestion peut être mécanique, enzymatique ou bien chimique par exemple.

A cet effet, dans un mode de réalisation particulier, le procédé objet de la présente invention comprend une étape de digestion de ladite portion de tissu biologique isolée, de sorte à en décellulariser au moins partiellement la matrice extracellulaire.

Dans un exemple de mise en œuvre particulier, l'étape de digestion peut par exemple être réalisée par mise en présence de la portion de tissu biologique isolée avec une solution de digestion comportant de la collagénase (digestion enzymatique). Cette digestion permet de couper le collagène qui maintient les cellules dans la matrice extracellulaire, et ainsi de libérer les cellules de la fraction stromale vasculaire, tout en éliminant une grande partie des cellules de la matrice restante. La matrice extracellulaire ainsi obtenue à l'issue de la digestion, puis d'une étape subséquente de centrifugation, est avantageusement riche en macromolécules telles que le collagène, l'élastine, les glycosaminoglycanes, les molécules d'adhésion cellulaire et des facteurs de croissance. Cette préparation de la matrice extracellulaire partiellement décellularisée, permet de favoriser l'adhésion sur ladite matrice, puis la prolifération, des cellules souches avec lesquelles elle est ultérieurement mise en contact et injectée en vue d'une réparation tissulaire. En effet, les molécules de la matrice extracellulaire fournissent un environnement favorable au développement cellulaire, à une homéostasie unique à chaque tissu biologique, en permettant l'adhésion cellulaire dès les premières minutes de mise en contact de la matrice avec des cellules souches. Plus le contact est long, plus le nombre de cellules qui adhèrent à la matrice est grand.

Dans un mode de mise en œuvre particulier du procédé objet de la présente invention, le mélange de la matrice et des cellules souches est réalisé pendant une durée d'au moins cinq minutes, de préférence au moins 15 minutes. Dans un exemple de mise en œuvre, ce mélange est réalisé sans agitation.

Selon un deuxième aspect, la présente invention vise une composition destinée à être injectée au niveau d'un tissu biologique, susceptible d'être obtenue par un procédé objet de la présente invention. Cette composition comporte un mélange de cellules souches et d'une matrice choisie parmi une matrice extracellulaire obtenue à partir d'une portion de tissu biologique isolée et une matrice synthétique biocompatible hydrophile, de charge positive et poreuse, ladite matrice ayant été soumise à un traitement par hypoxie.

Cette composition permet avantageusement de réparer efficacement les lésions tissulaires, par injection au niveau du site biologique à réparer, tout en présentant un coût de préparation limité.

En complément du traitement hypoxique, d'autres traitements peuvent être appliqués à la matrice pour l'adapter au tissu receveur. Par exemple, en établissant des liaisons covalentes avec des peptides d'adhésion cellulaire, et/ou en appliquant des contraintes mécaniques au niveau de la lésion tissulaire (gravité pour l'os, étirement, cisaillement pour le tendon), et/ou en réalisant des expansions cellulaires des cellules souches. Ainsi il est possible de viser différentes applications thérapeutiques pour la composition obtenue par le procédé objet de la présente invention. Par exemple, il est possible de réaliser une régénération osseuse par injection d'une composition obtenue directement par le procédé objet de la présente invention, comprenant des cellules souches (par exemple des AMSC) concentrées dans une matrice ostéoconductrice, synthétique ou non, au niveau des os longs pour l'aide à la consolidation ou pour le comblement de perte de substance osseuse de taille critique. On entend par taille critique, la taille à partir de laquelle l'os est incapable de se régénérer seul.

Dans un mode de réalisation particulier de la composition, ladite matrice et lesdites cellules souches ont été soumises à un traitement par hypoxie.

Selon un troisième aspect, la présente invention vise la composition objet de la présente invention pour son utilisation pour la réparation tissulaire, notamment la régénération et/ou la consolidation d'un tissu biologique, chez le mammifère. Comme exposé précédemment, ce tissu biologique peut être un tissu osseux, adipeux, tel qu'un tissu cutané, un tissu tendineux, etc...

Selon un quatrième aspect, la présente invention vise un kit de consommables pour la mise en œuvre d'un procédé objet de la présente invention, comportant au moins une solution hypoxique apte à générer une hypoxie d'une matrice extracellulaire obtenue à partir d'une portion de tissu biologique isolée et au moins une solution de digestion apte à décellulariser au moins partiellement ladite matrice extracellulaire.

Dans un mode de réalisation particulier, au moins un des éléments du kit de consommable comporte au moins un code d'identification dudit élément. Un tel code d'identification peut être par exemple un code-barres ou bien un QR code. DE tels codes d'identification sont identifiables par un dispositif d'identification automatique tel qu'un lecteur optique pour faciliter la mise en œuvre du procédé selon l'invention par un opérateur.

## Présentation des figures

L'invention sera mieux comprise à la lecture de la description suivante, donnée à titre d'exemple nullement limitatif, et faite en se référant aux figures qui représentent :

- Figure 1 : illustre les étapes du procédé de préparation de composition pour la réparation tissulaire selon un mode de réalisation de l'invention.
- Figure 2 : montre une image obtenue au microscope à fluorescence d'une coupe d'une matrice extracellulaire de tissu adipeux non traitée avec une solution hypoxique, et mélangée avec des cellules AMSC pendant 3 jours (contrôle).
- Figure 3 : montre une image obtenue au microscope à fluorescence d'une coupe d'une matrice extracellulaire de tissu adipeux traitée avec une solution hypoxique selon le procédé objet de la présente invention et mélangée avec des cellules AMSC pendant 3 jours.
- La figure 4 : montre une image obtenue au microscope à fluorescence d'une coupe de matrice synthétique non traitée avec la solution hypoxique et sur laquelle les cellules AMSC marquées GFP ont été ensemencées et cultivées pendant 2 jours.
- La figure 5 : montre une image obtenue au microscope à fluorescence d'une coupe de matrice synthétique traitée avec la solution hypoxique selon le procédé et sur laquelle les cellules AMSC marquées GFP ont été ensemencées et cultivées pendant 2 jours.

## Description détaillée de l'invention

De manière plus générale, la portée de la présente invention ne se limite pas aux modes de réalisation décrits ci-dessus à titre d'exemples non limitatifs, mais s'étend au contraire à toutes les modifications à la portée de l'homme de l'art. Chaque caractéristique d'un mode de réalisation peut être mise en œuvre isolément ou combinée à toute autre caractéristique de tout autre mode de réalisation de manière avantageuse.

La présente invention porte sur un procédé de préparation d'une composition destinée à être injectée au niveau d'un tissu biologique pour une réparation tissulaire, par mélange de cellules souches et d'une matrice choisie parmi une matrice extracellulaire obtenue à partir d'une portion de tissu biologique isolée et une matrice synthétique biocompatible hydrophile, de charge positive et poreuse, dans lequel ladite matrice est soumise à un traitement par hypoxie avant ledit mélange. Les cellules souches peuvent également être soumises à un traitement par hypoxie avant le mélange avec la matrice.

On distingue plusieurs différentes cellules souches pouvant être utilisées pour la mise en œuvre du procédé objet de la présente invention.

On distingue d'abord trois types de cellules souches : les cellules souches embryonnaires, les cellules souches adultes (post natal) et les cellules souches IPS (« *Induced pluripotent stem cells* » en terminologie anglo-saxonne).

Les cellules souches embryonnaires utilisées dans la présente invention peuvent être choisies parmi les cellules souches totipotentes, multipotentes et pluripotentes. Elles sont de préférence obtenues par une technique non destructive de l'embryon.

Parmi les cellules souches adultes on compte notamment pour la présente invention les cellules souches hématopoïétiques (HSC pour « *hematopoietic stem cells* » en terminologie anglo-saxonne) et les cellules souches mésenchymateuses (MSC pour « *mesenchymal stem cells* » en terminologie anglo-saxonne). Les MSC peuvent par exemple être des cellules souches mésenchymateuses de tissu adipeux (AMSC pour « *adipose mesenchymal stem cells* » en terminologie anglo-saxonne), des cellules souches mésenchymateuses de moelle osseuse (BMSC pour « *bone marrow mesenchymal stem cells* » en terminologie anglo-saxonne) ou d'autres cellules souches mésenchymateuses extraites de pulpe dentaire, de bulbe capillaire, etc.

Les cellules souches pour la mise en œuvre du procédé objet de la présente invention sont choisies parmi les cellules souches ci-dessus. Dans des modes de réalisation particuliers, un mélange d'au moins deux ou plusieurs de ces différents types cellules souches est utilisé pour constituer les cellules souches servant à la mise en œuvre du procédé selon la présente invention.

La matrice utilisée dans le procédé objet de la présente invention peut être une matrice extracellulaire naturelle ou bien être une matrice synthétique biocompatible, hydrophile, de charge positive et poreuse. Lorsque la matrice utilisée est naturelle, elle est obtenue à partir d'une portion de tissu biologique isolée d'un organisme humain ou animal. Ce tissu biologique est de préférence choisi en fonction du tissu biologique pour la réparation tissulaire duquel la composition est destinée à être utilisée. Par exemple, pour une réparation du derme, il est possible d'utiliser une matrice extracellulaire obtenue à partir d'une portion isolée de tissu adipeux. De même, pour une réparation d'un tendon, il est possible d'utiliser une matrice extracellulaire obtenue à partir d'une portion isolée de tissu tendineux.

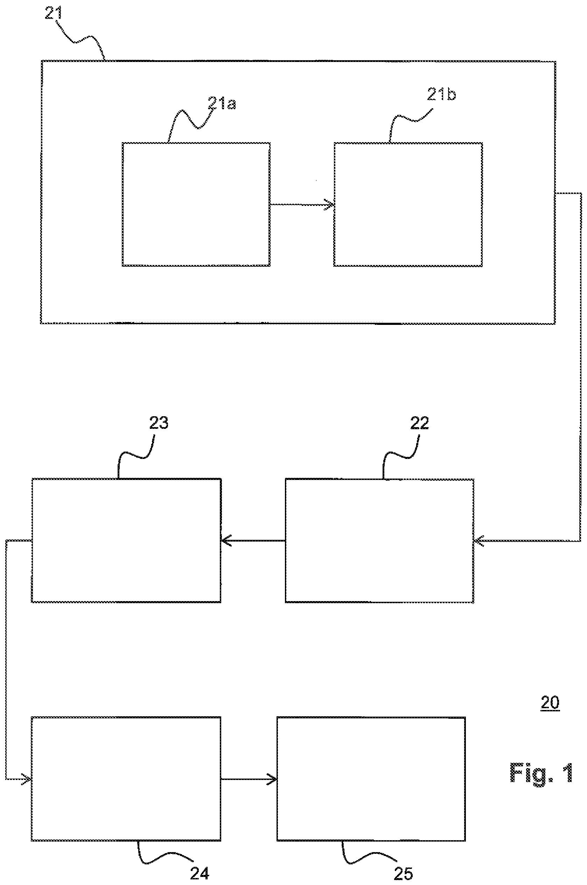

La figure 1 illustre un exemple non-limitatif d'un procédé **20** de préparation d'une composition destinée à être injectée au niveau d'un tissu biologique pour une réparation tissulaire selon l'invention, dans lequel la matrice extracellulaire est obtenue à partir d'une portion de tissu adipeux isolée d'un organisme humain ou animal.

La première étape **21,** consiste ainsi à séparer la matrice extracellulaire du tissu adipeux des cellules du tissu adipeux.

Cette première étape 21 peut comprendre une sous-étape **21a** de digestion du tissu adipeux avec une solution de digestion, suivie d'une sous-étape **21b** de centrifugation du tissu adipeux digéré, de sorte à séparer la matrice des cellules, pour obtenir une matrice partiellement décellularisée.

La digestion peut être enzymatique, mécanique ou bien chimique par exemple. Un exemple de digestion mécanique est la digestion du tissu adipeux par utilisation d'ultrasons.

La digestion peut se faire sous agitation.

La solution de digestion peut par exemple comporter de la collagénase (digestion enzymatique). Elle peut également comporter de la serum albumine bovine (BSA) ou de la serum albumine humaine (HSA), du tampon phosphate salin (PBS ou « *phosphate buffered saline* » en terminologie anglo-saxonne) sans calcium (Ca) ni magnésium (Mg). La collagénase coupe la plupart des liaisons collagènes entre les cellules du tissu adipeux et la matrice extracellulaire.

La sous-étape 21b permet quant à elle de séparer ladite matrice des cellules du tissu adipeux. La centrifugation de la sous-étape 21b permet d'obtenir différentes fractions, dont une comprend la matrice extracellulaire du tissu adipeux et une autre, correspondant au culot cellulaire, comprend des cellules de la fraction stromale vasculaire dudit tissu adipeux. On obtient également une troisième fraction lipidique

La matrice extracellulaire obtenue après digestion et centrifugation est complètement ou partiellement décellularisée.

Le procédé 20 peut comprendre ensuite une deuxième étape **22** d'au moins un lavage de la matrice extracellulaire avec une solution de lavage. La matrice extracellulaire est ensuite centrifugée de sorte à séparer la matrice de la solution de lavage et du reste de la solution de digestion. La fraction aqueuse comprenant la solution de lavage ainsi que des traces de solution de digestion, est éliminée après centrifugation.

La deuxième étape 22 d'au moins un lavage de la matrice extracellulaire peut être réalisée sous agitation. La solution de lavage peut être, par exemple, une solution comprenant du tampon phosphate salin (PBS) sans calcium (Ca) ni magnésium (Mg). La centrifugation peut être effectuée pendant environ 10 minutes à 300 g.

Dans des modes de réalisation particuliers de l'invention, la deuxième étape 22 comprend plusieurs lavages de la matrice extracellulaire et une centrifugation entre chaque lavage.

Dans une troisième étape **23,** la matrice extracellulaire du tissu adipeux récupérée à l'issue de la deuxième étape 22 est soumise à un traitement par hypoxie.

Cette troisième étape 23 est réalisée de préférence par mise en présence de la matrice avec une solution destinée à générer une hypoxie de la matrice dite solution hypoxique. Cette solution hypoxique peut par exemple comprendre du chlorure de cobalt ($CoCl_2$). La durée d'incubation de la matrice avec la solution hypoxique peut être par exemple de 15 minutes à plusieurs jours.

Dans une quatrième étape **24** facultative, la matrice est centrifugée de sorte à séparer la matrice de la solution hypoxique. Elle est suivie d'une opération d'élimination de la fraction aqueuse comprenant la solution hypoxique.

Enfin, dans une cinquième étape **25,** la matrice est mélangée avec des cellules souches.

Selon un mode de mise en œuvre particulier le procédé comprend à la suite de l'étape 21 et en parallèle d'au moins une des étapes 22 à 24, une étape (non représentée sur la figure 1) d'extraction des cellules souches mésenchymateuses du tissu adipeux (AMSC) pour la réalisation de l'étape 25, à partir des cellules du tissu adipeux obtenues à l'issue de la centrifugation réalisée à l'étape 21.

Dans un mode de mise en œuvre particulier du procédé, les cellules souches ont, préalablement au mélange 25, été soumises à un traitement par hypoxie.

*Selon un mode de réalisation particulier du procédé objet de la présente invention, il est mis en œuvre le protocole opératoire suivant :*

- étape 21a (digestion du tissu adipeux) : dans un tube, mise en présence du tissu adipeux avec une solution de digestion comportant de la collagénase de type I à 0,1%, de la BSA (2%), qsp PBS, V/V. Le tube est placé à 37°C pendant 30 minutes, dans un bain marie sous agitation forte (150 aller-retour/minute).
- étape 21b (séparation des éléments digérés) : l'ensemble du tissu adipeux digéré est centrifugé à 300 g pendant 10 minutes. La première phase en partant du bas du tube centrifugé est le culot cellulaire, la deuxième phase est constituée d'une fraction aqueuse, la troisième est constituée de la matrice extracellulaire et la quatrième est constituée de lipides relargués par des adipocytes. Cette centrifugation a pour fonction de séparer en fractions les différents éléments digérés. La fraction de la matrice extracellulaire est récupérée et isolée.
- étape 22 (lavage) : la matrice extracellulaire est lavée pendant 7 minutes, sous agitation, à température ambiante, avec une solution de PBS sans Ca ni Mg. La matrice est ensuite centrifugée 10 minutes à 300 g et la fraction aqueuse obtenue est éliminée afin de débarrasser la matrice extracellulaire de l'enzyme de digestion.
- étape 23 (hypoxie) : la matrice extracellulaire est mise en présence d'une solution de $CoCl_2$ à 150 $\mu$M dans du PBS, pendant 15 minutes. La solution de $CoCl_2$ (n°CAS = 7791-13-1) est préparée à partir de poudre de $CoCl_2$ hexahydraté ($Cl2 Co, 6 H_2O$).
- étape 24 (centrifugation) : le mélange de matrice et de solution de $CoCl_2$ est ensuite centrifugé à 300 g pendant 5 minutes, afin d'enlever le volume de $CoCl_2$.
- étape 25 (mélange matrice/cellules AMSC) : enfin, la matrice est mélangée avec des cellules souches mésenchymateuse de tissu adipeux (AMSC) pendant au moins 5 minutes.

*Variantes de mise en œuvre du procédé selon l'invention*

La concentration en collagénase de la solution de digestion enzymatique est généralement comprise entre 200 et 300 U/mL. La digestion peut être réalisée à une température comprise entre 34°C et 40°C, de préférence entre 35°C et 39 °C, préférentiellement à 37 °C. La digestion peut être réalisée dans une enceinte chauffante à sec, dans un équipement réalisant à la fois agitation et centrifugation, ou bien dans des équipements séparés, l'un réalisant l'agitation et l'autre la centrifugation.

La solution hypoxique peut être une solution de $CoCl_2$ ou bien une solution de type Deferoxamine (DFX) comprenant un ion fer chélateur, ou tout autre agent générant l'hypoxie. Le traitement par hypoxie de la matrice extracellulaire peut également être réalisé par le placement de ladite matrice dans un incubateur hypoxique, en pré-équilibrant l'incubateur en condition hypoxique (par exemple : 2% de dioxygène, 5% de dioxyde de carbone et 93% de diazote).

*Exemple d'obtention des cellules souches AMSC : à partir d'une fraction de tissu adipeux :*
Les cellules souches AMSC peuvent être obtenues de la manière suivante : après digestion enzymatique d'une fraction de tissu adipeux et centrifugation du produit de la digestion telles que décrites ci-dessus, la fraction du culot cellulaire est récupérée et isolée.

La fraction du culot cellulaire est ensuite centrifugée à 200 g pendant 10 minutes et le culot cellulaire obtenu est remis en suspension dans un tampon de lyse érythrocytaire (155 mmol/L $NH_4Cl$ ; 5,7 mmol/L $K_2HPO_4$ ; 0,1 mmol/L EDTA, pH 7,3) pendant 10 minutes.

Après des filtrations successives à travers des tamis respectivement de 100 $\mu$m, puis 70 $\mu$m et enfin 40 $\mu$m, les cellules AMSC sont remises en suspension dans une solution de PBS/2% de sérum de veau fœtal (SVF).

*Analyse de la viabilité de cellules souches dans une matrice extracellulaire traitée par hypoxie :*

1ère étape : infection des cellules avec le *lentivirus rLV.EF1.turboGFP.*

A partir d'une portion de tissu adipeux issue de liposuccion, des cellules AMSC isolées sont infectées par le lentivirus *rLV.EF1.turboGFP* (GFP : *« Green fluorescence Protein »* en terminologie anglo-saxonne) pour les rendre fluorescentes et donc différenciables par rapport aux cellules « résidentes » de la matrice extracellulaire (avec laquelle les cellules AMSC seront mélangées) qui n'auront pas été endommagées lors de l'étape de digestion du tissu adipeux (étape 21a du procédé de la présente invention).

Pour cela, les fractions SVF ont été ensemencées de manière similaire, dans 2 flasques, la veille de l'infection. Le jour J, les cellules d'une flasque sont comptées pour pouvoir déterminer le volume de vecteurs à ajouter aux cellules selon la formule suivante :

Volume vecteur viral (µl) = [nombre de cellules ensemencées/ titre vecteur viral (TU/ml)]/MOI x 1000,

avec MOI égal à 20 pour les SVF qui s'infectent bien.

TU : « *Transducing Unit »*
MOI : « *Multiplicity Of Infection »* (Nombre de virus attaquant la cible)

Pour une flasque d'un contenant de 10ml, sont incorporés :

- 9.95 mL de mélange comprenant le vecteur viral (à déterminer selon la formule citée plus haut en µl) + milieu complet (DMEM F12 + 10% de sérum de vœu fœtal) ;
- 50 µL de Polybrene à 800µg/ml (concentration finale 4µg/ml).

Incuber ce milieu de transduction avec les cellules AMSC pendant 16h maximum, et attendre 48h avant d'utiliser les cellules (c'est à ce moment que le maximum d'expression de la GFP apparait).

2ème étape : préparation de la matrice

A partir d'une portion isolée de tissu adipeux, on effectue une digestion dudit tissu adipeux, après lavage au PBS, pendant 30min à 37°C avec de la collagénase I à 300 U/ml. On effectue ensuite une centrifugation du tissu adipeux digéré à 1200 rpm pendant 10min et on récupère la fraction comportant la matrice extracellulaire.
Plusieurs lavages de la matrice sont effectués, notamment pour enlever un maximum de lipides de la matrice. Ces lavages sont effectués chacun avec du PBS pendant 2 min sous agitation et à 37°C. Une centrifugation de 5 min est effectuée après chaque lavage et la matrice extracellulaire est récupérée tandis que la phase aqueuse est éliminée.
Une fois que la matrice est lavée, elle est répartie dans 16 puits à quantités égales (200µl).
Ensuite, pour huit des puits, la matrice extracellulaire est mélangée avec une solution hypoxique à base de $CoCl_2$. Le temps de mise en présence de la solution de $CoCl_2$ avec la matrice est de 20min sous agitation à 37°C. La concentration initiale en $CoCl_2$ de la solution hypoxique est de 200 µM (finale à 50 µM). On effectue ensuite un lavage de la matrice au PBS après le traitement hypoxique.
Après les traitements des matrices, on effectue l'ensemencement des cellules AMSC transduites (mélange avec les matrices extracellulaires traitées et non traitées par hypoxie). Au total, on dispose de 1.1M de cellules AMSC. Les cellules sont reprises dans 8ml de milieu (DMEM F12 + 10% de sérum de veau fœtal) et 500µL de la suspension de cellules sont introduits dans chaque puits.
Les puits sont placés à l'étuve à 37°C.
Au bout de 3 jours, tous les puits sont regroupés, les puits traités ensemble et les non traités ensemble d'autre part, et centrifugés. Ensuite la matrice est fixée en étant déposée dans de l'OCT (« *optimal cutting temperature compound »* en terminologie anglo-saxonne), puis plongée dans de l'azote.
Des coupes fines des matrices extracellulaires sont réalisées. Les coupes de matrices sont colorées au DAPI et sont observées au microscope à fluorescence.

Résultats :

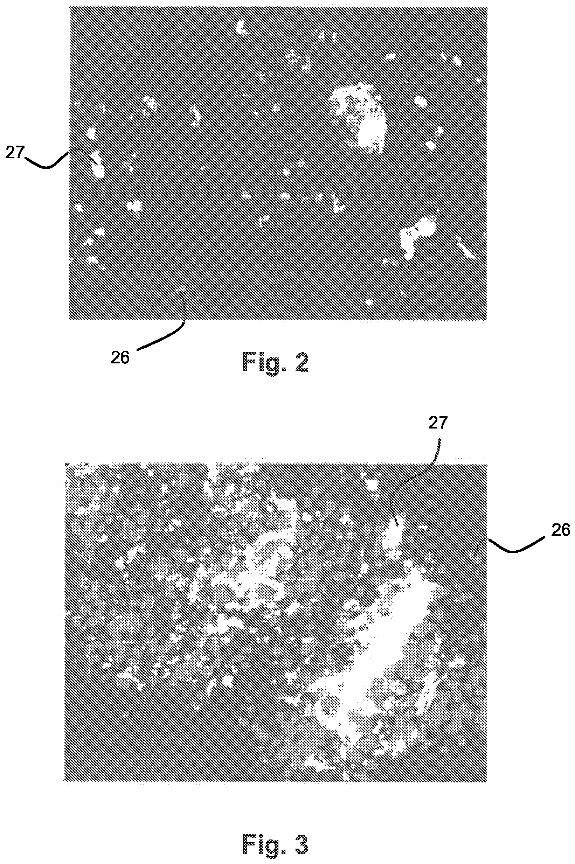

La figure 2, montre une image obtenue au microscope à fluorescence d'une coupe de la matrice extracellulaire de tissu adipeux non traitée avec la solution hypoxique et mélangée avec les cellules AMSC pendant 3 jours.
La figure 3, montre une image obtenue au microscope à fluorescence d'une coupe de la matrice extracellulaire de tissu adipeux traitée avec la solution hypoxique selon le procédé objet de la présente invention et mélangée avec les cellules AMSC pendant 3 jours.
Les cellules vivantes (26) dans la matrice sont colorées en bleu (colorant vital, visibles en gris sur les figures) et les cellules AMSC (27) qui ont été mélangées avec la matrice sont colorées en vert (visibles en blanc sur les figures).
Il apparait des figures 2 et 3 que la préparation de la matrice n'a pas détruit toutes les cellules qu'elle renfermait initialement. La matrice est partiellement décellularisée.
On observe que les cellules AMSC demeurent vivantes à l'issue de l'extraction et du mélange avec la matrice traitée ou non traitée avec la solution hypoxique.
Enfin, on constate qu'il y a bien plus d'AMSC vivantes au contact de la matrice traitée par hypoxie qu'au contact de la matrice non traitée.

Le temps d'adhésion cellulaire est compris entre 1 heure et 24 heures selon les types cellulaires et le substrat sur lequel les cellules adhèrent. Ici, l'expérience montre qu'au jour 3, des cellules AMSC vivantes ont adhéré à la matrice. Ce point est essentiel car c'est à partir de ce moment-là que les cellules peuvent ensuite proliférer et/ou se différencier. L'adhésion cellulaire est la base de la réussite de la régénération tissulaire recherchée.

Sans l'adhésion initiale des cellules AMSC, en condition de matrice non traitée par hypoxie, lors de leur injection au niveau d'un site tissulaire à réparer, ces cellules partent dans la circulation sanguine et sont rapidement éliminées. Il en résulte alors une inefficacité du procédé de réparation tissulaire.

Avantageusement, lorsque les cellules sont mélangées à la matrice traitée au préalable par hypoxie l'adhésion des cellules au site tissulaire à réparer est fortement favorisée du fait de leur bonne adhésion à la matrice.

*Analyse de la viabilité de cellules souches dans une matrice synthétique biocompatible, hydrophile, de charge positive, poreuse et traitée par hypoxie :*
L'étape 1 d'infection de cellules souches de l'analyse précédente est reproduite.

Ensuite, la matrice synthétique est déposée dans des puits. Elle se présente sous forme d'un disque de diamètre 13 mm et d'épaisseur 1 mm..

La matrice extracellulaire est mélangée avec une solution hypoxique à base de $CoCl_2$. Le temps de mise en présence de la solution de $CoCl_2$ avec la matrice est de 2 jours à 37°C. La concentration initiale en $CoCl_2$ de la solution hypoxique est de 200 $\mu M$ (finale à 50 $\mu M$). On effectue ensuite un lavage de la matrice au PBS après le traitement hypoxique.

Après le traitement de la matrice, on effectue l'ensemencement des cellules AMSC transduites. Pour cela, on mélange avec les matrices synthétiques traitées et non traitées par hypoxie 0.07M de cellules AMSC transduites par disque de matrice synthétique dans 50 $\mu l$ de milieu (DMEM F12 + 10% de serum de veau fœtal). Les puits sont placés à l'étuve à 37°C.

Au bout de 2 jours, chaque disque de matrice synthétique est fixé en étant déposé dans de l'OCT (« *optimal cutting temperature compound »* en terminologie anglo-saxonne), puis plongé dans de l'azote.

Des coupes fines des matrices synthétiques sont réalisées. Les coupes de matrices sont colorées au DAPI et sont observées au microscope à fluorescence.

Résultats :

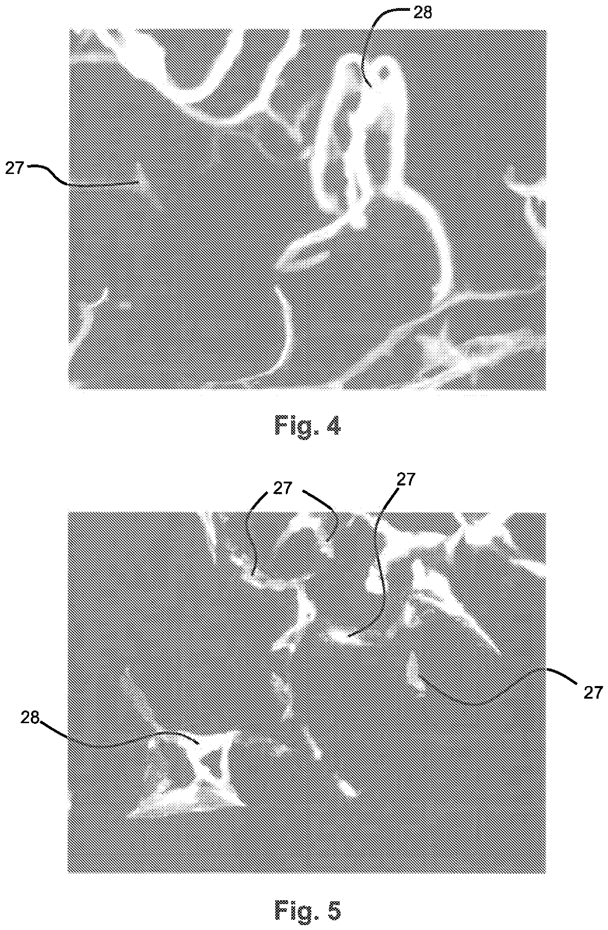

La figure 4 montre une image obtenue au microscope à fluorescence d'une coupe de matrice synthétique non traitée avec la solution hypoxique et sur laquelle les cellules AMSC marquées GFP ont été ensemencées et cultivées pendant 2 jours.

La figure 5 montre une image obtenue au microscope à fluorescence d'une coupe de matrice synthétique traitée avec la solution hypoxique selon le procédé et sur laquelle les cellules AMSC marquées GFP ont été ensemencées et cultivées pendant 2 jours.

Les cellules AMSC (27) vivantes sont colorées en vert (visibles en blanc sur les figures), on distingue le noyau et le cytoplasme.

La matrice synthétique (28) apparait colorée en vert plus intense et uniforme (visibles en blanc plus intense et uniforme sur les figures) que la coloration des cellules (27) ce qui permet de les différencier.

On observe que les cellules (27) demeurent vivantes sur la matrice synthétique (28), même si elles sont en nombre plus faible que sur la matrice extracellulaire. Le grossissement est ici aussi supérieur, ce qui limite le champ.

On constate qu'il y a plus de cellules vivantes (27) sur la matrice synthétique (28) traitée que sur la même matrice (28) non traitée.

Comme pour la matrice extracellulaire, on conclut que lorsque les cellules sont mélangées à la matrice synthétique traitée au préalable par hypoxie, l'adhésion des cellules au site tissulaire à réparer est fortement favorisée du fait de leur bonne adhésion à la matrice.

**Revendications**

1.   Procédé de préparation d'une composition destinée à être injectée au niveau d'un tissu biologique pour une réparation tissulaire, par mélange de cellules souches et d'une matrice choisie parmi une matrice extracellulaire obtenue à partir d'une portion de tissu biologique isolée et une matrice synthétique biocompatible hydrophile, de charge positive et poreuse, **caractérisé en ce que** ladite matrice est soumise à un traitement par hypoxie avant ledit mélange.

2.   Procédé de préparation selon la revendication 1 dans lequel les cellules souches sont soumises à un traitement par hypoxie avant le mélange.

3. Procédé de préparation selon l'une quelconque des revendications 1 à 2, dans lequel le traitement de la matrice par hypoxie comprend une étape de mise en présence de ladite matrice avec une solution destinée à générer une hypoxie de ladite matrice, dite solution hypoxique.

4. Procédé de préparation selon la revendication 3, dans lequel la solution hypoxique est une solution comprenant du chlorure de cobalt ($CoCl_2$).

5. Procédé de préparation selon l'une quelconque des revendications 1 à 4, comprenant une étape préalable d'extraction desdites cellules souches à partir d'une portion de tissu adipeux isolée d'un organisme humain ou animal.

6. Procédé de préparation selon l'une quelconque des revendications 1 à 5, comprenant une étape préalable d'obtention de la matrice extracellulaire à partir d'une portion de tissu biologique isolée d'un organisme humain ou animal, ledit tissu biologique étant du même type que le tissu biologique pour la réparation tissulaire duquel ladite composition est destinée à être utilisée.

7. Procédé de préparation selon la revendication 6, comprenant une étape de digestion de ladite portion de tissu biologique isolée, de sorte à décellulariser au moins partiellement ladite matrice extracellulaire.

8. Procédé de préparation selon la revendication 7, dans lequel ladite étape de digestion est réalisée par mise en présence de la portion de tissu biologique isolée avec une solution de digestion comprenant de la collagénase.

9. Procédé de préparation selon l'une quelconque des revendications 6 à 8, dans lequel la portion de tissu biologique isolée est une portion de tissu adipeux.

10. Procédé de préparation selon la revendication 9, comprenant une étape d'extraction des cellules souches à partir de la même portion de tissu adipeux servant à l'obtention de la matrice extracellulaire, ladite étape d'extraction des cellules souches et l'étape d'obtention de la matrice extracellulaire comprenant de préférence une même étape initiale de digestion de ladite portion de tissu adipeux isolée.

11. Procédé selon l'une quelconque des revendications 1 à 10, dans lequel le mélange de la matrice et des cellules souches est réalisé pendant une durée d'au moins cinq minutes, de préférence au moins quinze minutes.

12. Composition destinée à être injectée au niveau d'un tissu biologique, susceptible d'être obtenue par un procédé selon l'une quelconque des revendications 1 à 11, comportant un mélange de cellules souches et d'une matrice choisie parmi une matrice extracellulaire obtenue à partir d'une portion de tissu biologique isolée et une matrice synthétique biocompatible hydrophile, de charge positive et poreuse, ladite matrice ayant été soumise à un traitement par hypoxie.

13. Composition selon la revendication 12, dans laquelle ladite matrice et lesdites cellules souches ont été soumises à un traitement par hypoxie.

14. Composition selon l'une quelconque des revendications 12 à 13, pour son utilisation pour la réparation tissulaire, notamment la régénération et/ou la consolidation d'un tissu biologique, chez le mammifère.

15. Kit de consommables pour la mise en œuvre d'un procédé selon l'une quelconque des revendications 1 à 11, comportant au moins une solution hypoxique apte à générer une hypoxie d'une matrice extracellulaire obtenue à partir d'une portion de tissu biologique isolée et au moins une solution de digestion apte à décellulariser au moins partiellement ladite matrice extracellulaire.

16. Kit de consommables selon la revendication 15, dans lequel au moins un des éléments du kit de consommable comporte au moins un code d'identification dudit élément.

**Patentansprüche**

1. Verfahren zur Herstellung einer Zusammensetzung, die zur Injektion im Bereich eines biologischen Gewebes für eine Gewebereparatur bestimmt ist, durch Mischen von Stammzellen und einer Matrix, ausgewählt aus einer extrazellulären Matrix, erhalten aus einem isolierten Abschnitt eines biologischen Gewebes, und einer hydrophilen biologisch kompatiblen synthetischen Matrix, mit positiver Ladung und porös, **dadurch gekennzeichnet, dass** die Matrix vor dem Mischen einer Bearbeitung durch Hypoxie unterzogen wird.

2. Herstellungsverfahren nach Anspruch 1, wobei die Stammzellen vor dem Mischen einer Bearbeitung durch Hypoxie unterzogen werden.

3. Herstellungsverfahren nach einem der Ansprüche 1 bis 2, wobei die Bearbeitung der Matrix durch Hypoxie einen Schritt des Inanwesenheitversetzens der Matrix mit einer Lösung umfasst, die bestimmt ist, eine Hypoxie der Matrix zu erzeugen, bezeichnet als hypoxische Lösung.

4. Herstellungsverfahren nach Anspruch 3, wobei die hypoxische Lösung eine Lösung ist, die Cobaltchlorid ($CoCl_2$) umfasst.

5. Herstellungsverfahren nach einem der Ansprüche 1 bis 4, umfassend einen vorherigen Extraktionsschritt der Stammzellen aus einem Abschnitt eines adipösen Gewebes, der aus einem menschlichen oder tierischen Organismus isoliert wurde.

6. Herstellungsverfahren nach einem der Ansprüche 1 bis 5, umfassend einen vorherigen Schritt des Erhaltens der extrazellulären Matrix aus einem Abschnitt eines biologischen Gewebes, der aus einem menschlichen oder tierischen Organismus isoliert wurde, wobei das biologische Gewebe desselben Typs ist wie das biologische Gewebe für die Reparatur des Gewebes, für das die Zusammensetzung bestimmt ist, verwendet zu werden.

7. Herstellungsverfahren nach Anspruch 6, umfassend einen Digestionsschritt des isolierten Abschnitts eines biologischen Gewebes, so dass die extrazelluläre Matrix mindestens teilweise dezellularisiert wird.

8. Herstellungsverfahren nach Anspruch 7, wobei der Digestionsschritt durch Inanwesenheitversetzen des isolierten Abschnitts eines biologischen Gewebes mit einer Digestionslösung durchgeführt wird, die Collagenase umfasst.

9. Herstellungsverfahren nach einem der Ansprüche 6 bis 8, wobei der isolierte Abschnitt eines biologischen Gewebes ein Abschnitt eines adipösen Gewebes ist.

10. Herstellungsverfahren nach Anspruch 9, umfassend einen Extraktionsschritt der Stammzellen aus demselben Abschnitt eines adipösen Gewebes, das zur Gewinnung der extrazellulären Matrix dient, wobei der Extraktionsschritt der Stammzellen und der Schritt des Erhaltens der extrazellulären Matrix vorzugsweise einen gleichen Digestionsausgangschritt des isolierten Abschnitts eines adipösen Gewebes umfassen.

11. Verfahren nach einem der Ansprüche 1 bis 10, wobei das Mischen der Matrix und der Stammzellen während einer Dauer von mindestens fünf Minuten, vorzugsweise mindestens fünfzehn Minuten, durchgeführt wird.

12. Zusammensetzung, die zur Injektion im Bereich eines biologischen Gewebes bestimmt ist, die imstande ist, durch ein Verfahren nach einem der Ansprüche 1 bis 11 erhalten zu werden, aufweisend eine Mischung von Stammzellen und einer Matrix, ausgewählt aus einer extrazellulären Matrix, erhalten aus einem isolierten Abschnitt eines biologischen Gewebes, und einer hydrophilen biologisch kompatiblen synthetischen Matrix, mit positiver Ladung und porös, wobei die Matrix einer Bearbeitung durch Hypoxie unterzogen wurde.

13. Zusammensetzung nach Anspruch 12, wobei die Matrix und die Stammzellen einer Bearbeitung durch Hypoxie unterzogen wurden.

14. Zusammensetzung nach einem der Ansprüche 12 bis 13 für ihre Verwendung zur Gewebereparatur, insbesondere zur Regenerierung und/oder Konsolidierung eines biologischen Gewebes beim Säugetier.

15. Verbrauchsmaterialien-Set für die Durchführung eines Verfahrens nach einem der Ansprüche 1 bis 11, aufweisend mindestens eine hypoxische Lösung, die imstande ist, eine Hypoxie einer extrazellulären Matrix zu erzeugen, erhalten aus einem isolierten Abschnitt eines biologischen Gewebes, und mindestens eine Digestionslösung, die imstande ist, die extrazelluläre Matrix mindestens teilweise zu dezellularisieren.

16. Verbrauchsmaterialien-Set nach Anspruch 15, wobei mindestens eins der Elemente des Verbrauchsmaterialien-Sets mindestens einen Identifikationscode des Elements aufweist.

**Claims**

1.  Method for preparing a composition intended to be injected into a biological tissue for tissue repair, by mixing strain cells and a matrix chosen from an extracellular matrix obtained from an isolated portion of biological tissue and a hydrophilic biocompatible synthetic matrix, with a positive charge and porous, **characterised in that** said matrix is subjected to a treatment by hypoxia before said mixing.

2.  Preparation method according to claim 1, wherein the strain cells are subjected to a treatment by hypoxia before the mixing.

3.  Preparation method according to any one of claims 1 to 2, wherein the treatment of the matrix by hypoxia comprises a step of putting said matrix together with a solution intended to generate hypoxia of said matrix, referred to as a hypoxic solution.

4.  Preparation method according to claim 3, wherein the hypoxic solution is a solution comprising cobalt chloride ($CoCl_2$).

5.  Preparation method according to any one of claims 1 to 4, comprising a prior step of extracting said strain cells from a portion of adipose tissue isolated from a human or animal organism.

6.  Preparation method according to any one of claims 1 to 5, comprising a prior step of obtaining the extracellular matrix from a portion of biological tissue isolated from a human or animal organism, said biological tissue being of the same type as the biological tissue for the tissue repair of which said composition is intended to be used.

7.  Preparation method according to claim 6, comprising a step of digesting said isolated portion of biological tissue, so as to at least partially decellularise said extracellular matrix.

8.  Preparation method according to claim 7, wherein said digestion step is implemented by putting the isolated portion of biological tissue together with a digestion solution comprising collagenase.

9.  Preparation method according to any one of claims 6 to 8, wherein the isolated portion of biological tissue is a portion of adipose tissue.

10. Preparation method according to claim 9, comprising a step of extracting the strain cells from the same portion of adipose tissue used for obtaining the extracellular matrix, said step of extracting the strain cells and the step of obtaining the extracellular matrix preferably comprising the same initial step of digesting said isolated portion of adipose tissue.

11. Method according to any one of claims 1 to 10, wherein the matrix and the strain cells are mixed for a period of at least five minutes, preferably at least fifteen minutes.

12. Composition intended to be injected into a biological tissue, able to be obtained by a method according to any one of claims 1 to 11, including a mixing of strain cells and of a matrix selected from an extracellular matrix obtained from an isolated portion of biological tissue and a hydrophilic biocompatible synthetic matrix, with a positive charge and porous, said matrix having been subjected to treatment by hypoxia.

13. Composition according to claim 12, wherein said matrix and said strain cells have been subjected to a treatment by hypoxia.

14. Composition according to any one of claims 12 to 13, for use thereof for tissue repair, in particular regeneration and/or consolidation of a biological tissue, in a mammal.

15. Kit of consumables for implementing a method according to any one of claims 1 to 11, including at least one hypoxic solution able to generate hypoxia of an extracellular matrix obtained from an isolated portion of biological tissue and at least one digestion solution able to at least partially decellularise said extracellular matrix.

16. Kit of consumables according to claim 15, wherein at least one of the elements of the kit of consumables includes at least one code identifying said element.

Fig. 1

20

27

26

Fig. 2

27

26

Fig. 3

Fig. 4

Fig. 5

# EP 3 512 573 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2010330047 A

**Littérature non-brevet citée dans la description**

- **LIN et al.** Cavernous nerve repair with allogenic adipose matrix and autologous adipose-derived stem cells. *Urology,* Juin 2011, vol. 77 (6), 1509.e1-1509.e8
- **ZUK et al.** *Mol Biol Cell.,* Décembre 2002, vol. 13 (12), 4279-95
- **P. ZUK.** *Human Adipose Tissue Is a Source of Multipotent Stem Cells,* 2002